# EUROPEAN PATENT SPECIFICATION

(11) **EP 0 556 239 B1**
(45) Date of publication and mention of the grant of the patent: **30.08.1995**
(21) Application number: 91919478.7
(22) Date of filing: 07.11.1991
(51) Int. Cl.: A61K 9/12

(54) **AEROSOL CONTAINING MEDICAMENTS**
AEROSOLE DIE ARZNEIMITTEL ENTHALTEN
AEROSOL CONTENANT DES MEDICAMENTS

(30) Priority: 09.11.1990 GB 9024366
(43) Date of publication of application: 25.08.1993
(73) Proprietor: GLAXO GROUP LIMITED, Greenford, Middlesex UB6 0NN (GB)
(72) Inventor: TAYLOR, Anthony James, Ware, Hertfordshire SG12 0DG (GB); BURNELL, Patricia Kwong Phieu, Ware, Hertfordshire SG12 0DG (GB)
(74) Representative: Filler, Wendy Anne, Dr.
(86) International application number: GB9101961
(87) International publication number: WO9208447

(56) References cited:
- EP-A- 0 372 777
- WO-A-91/11173
- WO-A-91/11495

## Description

This invention relates to aerosol formulations of use in the administration of medicaments by inhalation.

The use of aerosols to administer medicaments has been known for several decades. Such aerosols generally comprise the medicament, one or more chlorofluorocarbon propellants and either a surfactant or a solvent, such as ethanol.

The most commonly used aerosol propellants for medicaments have been Freon 11 (CCl₃F) in admixture with Freon 12 (CCl₂F₂) and Freon 114 (CF₂Cl.CF₂Cl). However these propellants are now believed to provoke the degradation of stratospheric ozone and there is thus a need to provide aerosol formulations for medicaments which employ so called "ozone-friendly" propellants.

A class of propellants which are believed to have minimal ozone-depleting effects in comparison to conventional chlorofluorocarbons comprise hydrogen-containing chlorofluorocarbons and fluorocarbons; medicinal aerosol formulations using such propellant systems are disclosed in, for example, EP 0372777. EP 0372777 requires the use of 1,1,1,2-tetrafluoroethane in combination with both a cosolvent having greater polarity than 1,1,1,2-tetrafluoroethane (e.g. an alcohol or a lower alkane) and a surfactant in order to achieve a stable formulation of a medicament powder. In particular it is noted in the specification at page 3, line 7 that "it has been found that the use of Propellant 134a (1,1,1,2-tetrafluoroethane) and drug as a binary mixture or in combination with a conventional surfactant such as sorbitan trioleate does not provide formulations having suitable properties for use with pressurised inhalers".

We have now surprisingly found that, in contradistinction to this teaching, it is in fact possible to obtain stable dispersions of certain finely-powdered medicaments together with certain surfactants in hydrogen-containing fluorocarbon or chlorofluorocarbon propellants such as 1,1,1,2-tetrafluoroethane if the surfactant is present as a dry coating on the particles of medicament. More particularly, such stable dispersions may be formed where the medicament is selected from salmeterol, fluticasone esters, 4-amino-3,5-dichloro-α-[[[6-[2-(2-pyridinyl)ethoxy]hexyl] amino]methyl]benzenemethanol, and physiologically acceptable salts and solvates thereof. This is in contrast to the procedure of EP 0372777, where the medicament and surfactant are simultaneously homogenised, e.g. in ethanol, prior to addition of the propellant.

There is thus provided an aerosol formulation comprising (A) a medicament selected from the group comprising salmeterol, fluticasone esters, 4-amino-3,5-dichloro-α-[[[6-[2-(2-pyridinyl)ethoxy]hexyl]amino]methyl]benzenemethanol, and physiologically acceptable salts and solvates thereof in particulate form and having a surface-coating of a surfactant; and (B) a hydrogen-containing fluorocarbon or chlorofluorocarbon propellant.

The propellants for use in the invention may be any hydrogen-containing fluorocarbon or chlorofluorocarbon or mixtures thereof having a sufficient vapour pressure to render them effective as propellants. Such propellants include for example C₁₋₄hydrogen-containing fluorocarbons and chlorofluorocarbons such as CH₂ClF, CClF₂-CHClF, CF₃-CHClF, CHF₂-CClF₂, CHClF-CHF₂, CF₃-CH₂Cl, CHF₂-CHF₂, CF₃-CH₂F, CClF₂-CH₃, CHF₂-CH₃ and CF₃CHFCF₃.

Where mixtures of the hydrogen-containing fluorocarbons or chlorofluorocarbons are employed they may be mixtures of the above identified compounds or mixtures, preferably binary mixtures, with other hydrogen-containing fluorocarbons or chlorofluorocarbons for example CHClF₂, CH₂F₂ and CF₃CH₃.

The propellant may additionally contain a volatile saturated hydrocarbon for example n-butane, isobutane, pentane and isopentane. Preferably a single hydrogen-containing fluorocarbon or chlorofluorocarbon is employed as the propellant. Preferably the propellant will be a non-solvent for the medicament. Particularly preferred as propellants are 1,1,1,2-tetrafluoroethane (CF₃.CH₂F) and 1,1,1,2,3,3,3-heptafluoro-n-propane (CF₃.CHF.CF₃).

It is desirable that the formulations of the invention contain no components which may provoke the degradation of stratospheric ozone. In particular it is desirable that the formulations are substantially free of chlorofluorocarbons such as CCl₃F, CCl₂F₂ and CF₃CCl₃.

It is further desirable that the formulations of the invention are substantially free of liquid components of higher polarity than the propellant employed. In particular formulations which are free of alcohols such as ethanol are preferable.

Polarity may be determined for example, by the method described in European Patent Application Publication No. 0327777.

As used herein "substantially free" means less than 1% w/w based upon the hydrogen-containing fluorocarbon or chlorofluorocarbon, in particular less than 0.5% for example 0.1% or less.

Where appropriate the medicaments may be used in the form of salts (e.g. as alkali metal or amine salts or as acid addition salts) or as eaters (e.g. lower alkyl eaters) to optimise the activity and/or stability of the medicament and/or to minimise the solubility of the medicament in the propellant.

For use in the formulations of the present invention, salmeterol will preferably be in the form of its 1-hydroxy-2-naphthoate salt, the fluticasone ester will preferably be the propionate, and 4-amino-3,5-dichloro-α-[[[6-[2-(2-pyridinyl)ethoxy] hexyl]amino]methyl]benzenemethanol will preferably be in the form of the (R) enantiomer.

The surfactants for use in the invention will have no affinity for the propellant (that is to say they will contain no groups which have affinity with the propellant).

The surfactants must be physiologically acceptable upon administration by inhalation. Surfactants within this category include materials such as benzalkonium chloride, lecithin, oleic acid and sorbitan trioleate (Span ^{R} 85).

The use of substantially non-ionic surfactants which have reasonable solubility in substantially non-polar solvents is frequently advantageous since it facilitates coating of the medicament particles using solutions of surfactant in non-polar solvents in which the medicament has limited or minimal solubility.

Thus according to a further aspect of the invention the surfactant-coated medicament may be prepared by slurrying particulate (e.g. micronised) medicament with a solution of a surfactant such as lecithin in a substantially non-polar solvent (e.g. a lower alkane such as isopentane or a chlorofluorocarbon such as trichlorofluoromethane), optionally homogenising the slurry (e.g. by sonication), removing the solvent and if necessary simultaneously and/or subsequently breaking up the resulting solid cake. The thus-obtained surfactant-coated particulate medicaments are novel and form a further feature of the invention.

The formulations of the invention may be prepared by dispersing the surface-coated medicament, obtained as described above, in the chosen propellant in an appropriate aerosol container, e.g. with the aid of sonication.

The particle size of the finely-powdered medicament should be such as to permit inhalation of substantially all of the medicament into the bronchial system upon administration of the aerosol formulation and will thus be less than 100 »m, desirably less than 20 »m, and preferably in the range 2-10 »m, e.g. 2-5 »m.

The amount of surfactant employed in coating the particulate medicament is desirably in the range 0.01 - 10.0% w/w, preferably 0.05 - 5.0% w/w, relative to the medicament, and may advantageously be chosen such that a substantially monomolecular coating of surfactant is formed. The final aerosol formulation desirably contains 0.005 - 5.0% w/w, preferably 0.01 - 1.0% w/w, of coated medicament relative to the total weight of the formulation.

The following non-limitative Examples serve to illustrate the invention.

### EXAMPLE 1

### (A) Preparation of Lecithin-coated Salmeterol Hydroxynaphthoate

(a) Lecithin (Epikuron 145V - 3.65mg) was dissolved in a small amount of isopentane and the resulting solution was added to micronised salmeterol hydroxynaphthoate (0.5g). Further isopentane (7.0g total) was added to form a slurry, which was sonicated for 3 minutes. The resulting suspension was dried by evaporating the isopentane at ambient temperature in a fume cupboard, whereafter the resulting dried plug was roughly broken up and then dried further in a vacuum oven. The thus-obtained product was further broken up using a mortar and pestle to yield lecithin-coated salmeterol hydroxynaphthoate containing 0.73% w/w of lecithin relative to the salmeterol hydroxynaphthoate.
(b) The above procedure was repeated except that 6.10mg of lecithin was employed, whereby a coated product containing 1.22% w/w of lecithin relative to the salmeterol hydroxynaphthoate was obtained.
(c) The above procedure was again repeated except that 7.80mg of lecithin was employed, thereby yielding a coated product containing 1.56% w/w of lecithin relative to the salmeterol hydroxynaphthoate.

### (B) Formulation of Lecithin-coated Salmeterol Hydroxynaphthoate in 1,1,1,2-Tetrafluoroethane

Samples of each of the products of Example 1A (a)-(c) (9.1mg) were weighed into aerosol cans. 1,1,1,2-Tetrafluoroethane (18.2g - 99.5% w/w of total fill weight) was added to each can, whereafter suitable metering valves were crimped onto the cans, which were then each sonicated for 5 minutes. The resulting aerosols contained salmeterol in an amount equivalent to 240 actuations at 25»g per actuation.

### Example 2

### (A) Preparation of lecithin-coated fluticasone propionate

Lecithin (Epikuron 145v - 2.5mg) was dissolved in a small amount of isopentane and the resulting solution was added to micronised fluticasone propionate (0.5g). Further isopentane (20ml) was added to form a slurry, which was sonicated for 3 minutes. The resulting suspension was dried by evaporating the isopentane at ambient temperature in a fume cupboard, whereafter the resulting dried plug was roughly broken up and then dried further in a vacuum oven. The thus-obtained product was further broken up using a mortar and pestle to yield lecithin-coated fluticasone propionate containing 0.5% ^{w}/_{w} of lecithin relative to the fluticasone propionate.

### (B) Formulation of lecithin-coated fluticasone propionate in 1,1,1,2-tetrafluoroethane

A sample of the product of Example 2(A) (9.1mg) was weighed into aerosol cans, 1,1,1,2-Tetrafluoroethane (18.2g - 99.5% w/w of total fill weight) was added to each can, whereafter suitable metering valves were crimped onto the cans, which were then each sonicated for 5 minutes. The resulting aerosols contained fluticasone propionate in an amount equivalent to 240 actuations at 25»g per actuation.

### Example 3

### (A) Preparation of Oleic Acid-coated Salmeterol Hydroxynaphthoate

Oleic acid (10mg) was dissolved in a small amount of isopentane and the resulting solution was added to micronised salmeterol hydroxynaphthoate (1.0g). Further isopentane (25ml total) was added to form a slurry, which was sonicated for 3 minutes. The resulting suspension was dried by evaporating the isopentane at ambient temperature in a fume cupboard, whereafter the resulting dried plug was roughly broken up and then dried further in a vacuum oven. The thus-obtained product was further broken up using a mortar and pestle to yield oleic acid-coated salmeterol hydroxynaphthoate containing 1.0% w/w of oleic acid relative to the salmeterol hydroxynaphthoate.

### (B) Formulation of Oleic acid-coated Salmeterol Hydroxynaphthoate in 1,1,1,2-Tetrafluoroethane

Samples of the product of Example 3A (9.1mg) were weighed into aerosol cans. 1,1,1,2-Tetrafluoroethane (18.2g - 99.5% w/w of total fill weight) was added to each can, whereafter suitable metering valves were crimped onto the cans, which were then each sonicated for 5 minutes. The resulting aerosols contained salmeterol in an amount equivalent to 240 actuations at 25»g per actuation.

### Example 4

### (A) Preparation of Sorbitan Trioleate-coated Salmeterol Hydroxynaphthoate

Sorbitan trioleate (Span 85-10mg) was dissolved in a small amount of isopentane and the resulting solution was added to micronised salmeterol hydroxynaphthoate (1.0g). Further isopentane (25ml total) was added to form a slurry, which was sonicated for 3 minutes. The resulting suspension was dried by evaporating the isopentane at ambient temperature in a fume cupboard, whereafter the resulting dried plug was roughly broken up and then dried further in a vacuum oven. The thus-obtained product was further broken up using a mortar and pestle to yield sorbitan trioleate-coated salmeterol hydroxynaphthoate containing 1.0% w/w of sorbitan trioleate relative to the salmeterol hydroxynaphthoate.

### (B) Formulation of Sorbitan Trioleate-coated Salmeterol Hydroxynaphthoate in 1,1,1,2-Tetrafluoroethane

Samples of the product of Example 4A (9.1mg) were weighed into aerosol cans. 1,1,1,2-Tetrafluoroethane (18.2g - 99.5% w/w of total fill weight) was added to each can, whereafter suitable metering valves were crimped onto the cans, which were then each sonicated for 5 minutes. The resulting aerosols contained salmeterol in an amount equivalent to 240 actuations at 25»g per actuation.

## Claims

1. An aerosol formulation comprising :
(A) a medicament selected from the group comprising salmeterol, fluticasone esters, 4-amino-3,5-dichloro-α-[[[6-[2-(2-pyridinyl)ethoxy]hexyl]amino]methyl] benzenemethanol and physiologically acceptable salts and solvates thereof in particulate form and having a surface coating of a surfactant; and
(B) a hydrogen-containing fluorocarbon or chlorofluorocarbon propellant.

2. A formulation as claimed in Claim 1 wherein the propellant comprises 1,1,1,2-tetrafluoroethane or 1,1,1,2,3,3,3-heptafluoropropane.

3. A formulation as claimed in Claim 1 wherein the propellant comprises 1,1,1,2-tetrafluoroethane.

4. A formulation as claimed in any one of Claims 1 to 3 which is substantially free of chlorofluorocarbons.

5. A formulation as claimed in any one of Claims 1 to 4 which is substantially free of liquid components of higher polarity than the propellant.

6. A formulation as claimed in any one of Claims 1 to 5 wherein the coated medicament has a particle size of less than 100 »m.

7. A formulation as claimed in any one of Claims 1 to 6 wherein the surfactant is present in an amount of from 0.01 to 10% w/w based on the medicament.

8. A formulation as claimed in any one of Claims 1 to 7 wherein the surfactant is selected from benzalkonium chloride, lecithin, oleic acid and sorbitan trioleate.

9. A formulation as claimed in any one of Claims 1 to 8 wherein the surfactant-coated medicament is present in an amount of 0.005-5% w/w based upon the total weight of the medicament.

10. A formulation as claimed in any one of Claims 1 to 9 wherein the medicament is salmeterol in the form of its 1-hydroxy-2-naphthoate salt.

11. A formulation as claimed in any one of Claims 1 to 9 wherein the medicament is fluticasone propionate.

12. A formulation as claimed in any one of Claims 1 to 9 wherein the medicament is the (R) enantiomer of 4-amino-3,5-dichloro-α-[[[6-[2-(2-pyridinyl)ethoxy]hexyl]amino] methyl]benzenemethanol.

13. A method for the preparation of an aerosol formulation comprising dispersing a surface-coated medicament selected from the group comprising salmeterol, fluticasone esters, 4-amino-3,5-dichloro-α-[[[6-[2-(2-pyridinyl)ethoxy]hexyl]amino]methyl] benzenemethanol and physiologically acceptable salts and solvates thereof in a hydrogen-containing fluorocarbon or chlorofluorocarbon propellant in an aerosol container.

14. A method as claimed in Claim 13 wherein the surface-coated medicament is obtained by slurrying particulate medicament with a solution of a surfactant in a substantially non-polar solvent and then removing the solvent.

## Patentansprüche

1. Aerosolzubereitung, enthaltend
(A) ein Arzneimittel, ausgewählt aus der Gruppe: umfassend Salmeterol, Fluticasonester, 4-Amino-3,5-dichlor-α-[[[6-[2-(2-pyridinyl)ethoxy]hexyl]amino]methyl]benzolmethanol und die physiologisch annehmbaren Salze und Solvate davon, in teilchenförmiger Form mit einem Oberflächenüberzug eines oberflächenaktiven Mittels; und
(B) ein Wasserstoff-enthaltendes Fluorkohlenwasserstoff- oder Chlorfluorkohlenwasserstoff-Treibmittel.

2. Zubereitung nach Anspruch 1, dadurch **gekennzeichnet,** daß das Treibmittel 1,1,1,2-Tetrafluorethan oder 1,1,1,2,3,3,3-Heptafluorpropan umfaßt.

3. Zubereitung nach Anspruch 1, dadurch **gekennzeichnet,** daß das Treibmittel 1,1,1,2-Tetrafluorethan umfaßt.

4. Zubereitung nach einem der Ansprüche 1 bis 3, dadurch **gekennzeichnet,** daß sie im wesentlichen von Chlorfluorkohlenwasserstoffen frei ist.

5. Zubereitung nach einem der Ansprüche 1 bis 4, dadurch **gekennzeichnet,** daß sie im wesentlichen von flüssigen Komponenten mit höherer Polarität als das Treibmittel frei ist.

6. Zubereitung nach einem der Ansprüche 1 bis 5, dadurch **gekennzeichnet,** daß das beschichtete Arzneimittel eine Teilchengröße von weniger als 100 »m hat.

7. Zubereitung nach einem der Ansprüche 1 bis 6, dadurch **gekennzeichnet,** daß das oberflächenaktive Mittel in einer Menge von 0,01 bis 10% Gew./Gew., bezogen auf das Arzneimittel, vorhanden ist.

8. Zubereitung nach einem der Ansprüche 1 bis 7, dadurch **gekennzeichnet,** daß das oberflächenaktive Mittel aus Benzalkoniumchlorid, Lecithin, Ölsäure und Sorbitantrioleat ausgewählt ist.

9. Zubereitung nach einem der Ansprüche 1 bis 8, dadurch **gekennzeichnet,** daß das mit dem oberflächenaktiven Mittel beschichtete Arzneimittel in einer Menge von 0,005 bis 5% Gew./Gew., bezogen auf das Gesamtgewicht des Arzneimittels, vorhanden ist.

10. Zubereitung nach einem der Ansprüche 1 bis 9, dadurch **gekennzeichnet,** daß das Arzneimittel Salmeterol in der Form seines 1-Hydroxy-2-naphthoatsalzes ist.

11. Zubereitung nach einem der Ansprüche 1 bis 9, dadurch **gekennzeichnet,** daß das Arzneimittel Fluticasonpropionat ist.

12. Zubereitung nach einem der Ansprüche 1 bis 9, dadurch **gekennzeichnet,** daß das Arzneimittel das (R)-Enantiomere von 4-Amino-3,5-dichlor-α-[[[6-[2-(2-pyridinyl)ethoxy]hexyl]amino]methyl]benzolmethanol ist.

13. Verfahren zur Herstellung einer Aerosolzubereitung, umfassend die Dispergierung eines oberflächenbeschichteten Arzneimittels, ausgewählt aus der Gruppe, umfassend Salmeterol, Fluticasonester, 4-Amino-3,5-dichlor-α-[[[6-[2-(2-pyridinyl)ethoxy]hexyl]amino]methyl]benzolmethanol und die physiologisch annehmbaren Salze und Solvate davon, in einem Wasserstoff-enthaltenden Fluorkohlenwasserstoff- oder Chlorfluorkohlenwasserstoff-Treibmittel in einem Aerosolbehälter.

14. Verfahren nach Anspruch 13, dadurch **gekennzeichnet,** daß das oberflächenbeschichtete Arzneimittel durch Aufschlämmung des teilchenförmigen Arzneimittels mit einer Lösung eines oberflächenaktiven Mittels in einem im wesentlichen nichtpolaren Lösungsmittel und durch anschließende Entfernung des Lösungsmittels erhalten worden ist.

## Revendications

1. Composition d'aérosol comprenant :
(A) un médicament choisi dans le groupe formé par le salmétérol, des esters de la fluticasone, le 4-amino-3,5-dichloro-α-[[[6-[2-(2-pyridinyl)éthoxy]hexyl]amino]méthyl]benzèneméthanol, et les sels et solvates physiologiquement acceptables de ces composés, sous forme particulaire et comportant un revêtement en surface d'un surfactif, et
(B) un propulseur constitué par un fluorocarbone ou chlorofluorocarbone contenant de l'hydrogène.

2. Composition suivant la revendication 1, caractérisée en ce que le propulseur est constitué par le 1,1,1,2-tétrafluoréthane ou le 1,1,1,2,3,3,3-heptafluoropropane.

3. Composition suivant la revendication 1, caractérisée en ce que le propulseur est constitué de 1,1,1,2-tétrafluoréthane.

4. Composition suivant l'une quelconque des revendications 1 à 3, caractérisée en ce qu'elle est sensiblement dépourvue de chlorofluorocarbones.

5. Composition suivant l'une quelconque des revendications 1 à 4, caractérisée en ce qu'elle est sensiblement dépourvue de composants liquides de polarité supérieure à celle du propulseur.

6. Composition suivant l'une quelconque des revendications 1 à 5, caractérisée en ce que le médicament enrobé possède un calibre des particules inférieur à 100 »m.

7. Composition suivant l'une quelconque des revendications 1 à 6, caractérisée en ce que le surfactif est présent en une proportion de 0,01 à 10% p/p sur base du médicament.

8. Composition suivant l'une quelconque des revendications 1 à 7, caractérisée en ce que le surfactif est choisi parmi le chlorure de benzalconium, la lécithine, l'acide oléique et le trioléate de sorbitan.

9. Composition suivant l'une quelconque des revendications 1 à 8, caractérisée en ce que le médicament enrobé de surfactif est présent en une proportion de 0,005 à 5% p/p sur base du poids total du médicament.

10. Composition suivant l'une quelconque des revendications 1 à 9, caractérisée en ce que le médicament est le salmétérol sous forme de son sel du type 1-hydroxy-2-naphtoate.

11. Composition suivant l'une quelconque des revendications 1 à 9, caractérisée en ce que le médicament est le propionate de fluticasone.

12. Composition suivant l'une quelconque des revendications 1 à 9, caractérisée en ce que le médicament est l'énantiomère (R) du 4-amino-3,5-dichloro-α-[[[6-[2-(2-pyriodinyl)éthoxy]hexyl]amino]méthyl]benzèneéthanol.

13. Procédé de préparation d'une composition d'aérosol comprenant la dispersion d'un médicament revêtu en surface, choisi dans le groupe formé par le salmétérol, des esters de fluticasone, le 4-amino-3,5-dichloro-α-[[[6-[2-(2-pyridinyl)éthoxy]hexyl]amino]méthyl]benzèneméthanol et les sels et solvates physiologiquement acceptables de ces composés, dans un fluorocarbone ou un chlorofluorocarbone contenant l'hydrogène dans un récipient pour aérosol.

14. Procédé selon la revendication 13, caractérisé en ce que le médicament revêtu en surface s'obtient en mettant le médicament particulaire en suspension avec une solution d'un surfactif dans un solvant sensiblement apolaire et en chassant ensuite le solvant.
